# EUROPEAN PATENT APPLICATION

(11) **EP 4 230 726 A1**
(43) Date of publication of application: **23.08.2023**
(21) Application number: 21895082.2
(22) Date of filing: 17.11.2021
(51) Int. Cl.: C12N 5/071, C12N 5/00, C12M 3/00

(54) **PANCREATIC EXTRACELLULAR MATRIX-DERIVED SUPPORT FOR PANCREATIC ORGANOID CULTURE AND TRANSPLANTATION, AND METHOD FOR PREPARING SAME**

(30) Priority: 17.11.2020 KR 20200153718; 16.11.2021 KR 20210157356
(71) Applicant: Cellartgen Inc., Seoul 03722 (KR)
(72) Inventor: CHO, Seung Woo, Seoul 03722 (KR); KIM, Su Kyeom, Seoul 03726 (KR); CHOI, Yi Sun, Seoul 03722 (KR)
(74) Representative: Manitz Finsterwald Patent- und Rechtsanwaltspartnerschaft mbB
(86) International application number: PCT/KR2021/016826
(87) International publication number: WO 2022/108314

(57) **Abstract**

The present disclosure relates to a scaffold for culture and transplantation of a pancreatic organoid by using a decellularized pancreatic tissue (pancreas extracellular matrix; PEM).

## Description

### TECHNICAL FIELD

The present disclosure relates to a pancreas extracellular matrix-derived scaffold for culture and transplantation of a pancreatic organoid and a method of preparing the same.

### BACKGROUND

An organoid, which has recently attracted a lot of interest, is the technology that has been rapidly growing worldwide and can be applied as a tissue analogue to various clinical applications, such as drug screening, drug toxicity evaluation, disease modeling, cell therapeutic agent and tissue engineering. The organoid is a three-dimensional structure composed of various cells of a specific human organ and tissue and can implement complicated interactions between them. Therefore, it can be applied as a more accurate in vitro model platform than conventionally used drug evaluation models such as simple cell line models or animal models.

Various types of organoids exist for each organ in addition to the pancreas. Numerous research teams around the world researching these types of organoids are using Matrigel products as culture scaffolds to culture organoids. However, since Matrigel is extracted from mouse sarcoma tissue, it is difficult to maintain the quality of products uniformly and it is expensive and has a safety problem such as metastasis of animal pathogens and viruses. Therefore, Matrigel as an organoid culture system has a lot of problems to be solved. In particular, as a material derived from cancer tissue, it cannot provide an optimal tissue-specific microenvironment necessary for culturing a specific tissue organoid. There have been some studies on the development of polymer-based hydrogels to replace Matrigel, but no material has been reported that can replace Matrigel.

A pancreatic organoid is produced by extracting adult stem cells from a pancreatic tissue and culturing them or by differentiating pluripotent stem cells, such as human-induced pluripotent stem cells or embryonic stem cells into pancreatic progenitor cells and culturing them. Since Matrigel cannot implement a complex pancreatic tissue-specific microenvironment in vivo, the pancreatic organoid differentiation efficiency and function need to be improved. Accordingly, there is a desperate need for the development of a culture system for producing a more matured and functional pancreatic organoid.

Also, there is a need for an in vitro model platform for disease modeling research and drug testing to implement intractable pancreatic diseases, which result in a large amount of tissue loss and pancreatic failure such as acute and chronic pancreatitis, diabetes, and pancreatic cancer, in vitro and investigate the mechanisms thereof.

To solve the present technical problems in pancreatic organoid culture and its application technologies, in the present disclosure, a decellularized scaffold derived from a pancreatic tissue was prepared from the pancreatic tissue through decellularization, and a new platform using the decellularized scaffold for pancreatic organoid culture is proposed. The developed decellularized pancreatic tissue-derived hydrogel scaffold abundantly contains various pancreatic tissue-specific extracellular matrices and growth factors and thus improves differentiation, maturity, and functionality of a pancreatic organoid compared to Matrigel.

### DISCLOSURE OF THE INVENTION

### PROBLEMS TO BE SOLVED BY THE INVENTION

The present disclosure is conceived to obtain a large amount of decellularized tissue through a chemical treatment of a porcine pancreatic tissue, prepare a hydrogel scaffold based on the decellularized tissue and apply it to pancreatic organoid culture.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

An aspect of the present disclosure provides a scaffold for culture and transplantation of a pancreatic organoid, containing a pancreas extracellular matrix (PEM).

In an embodiment of the present disclosure, the PEM may be decellularized by treating a pancreatic tissue with a mixed solution of Triton X-100 and ammonium hydroxide.

In an embodiment of the present disclosure, a concentration of the PEM in the scaffold may be from 1 mg/ml to 10 mg/ml.

Another aspect of the present disclosure provides a method of preparing a scaffold for culture and transplantation of a pancreatic organoid, including: a process (1) of crushing an isolated pancreatic tissue; and a process (2) of treating the crushed pancreatic tissue with Triton X-100 and ammonium hydroxide for decellularization to prepare a decellularized PEM.

In an embodiment of the present disclosure, the method may further include, after the process (2), a process (3) of lyophilizing the decellularized PEM to prepare a lyophilized PEM.

In an embodiment of the present disclosure, the method may further include, after the process (3), a process (4) of forming a scaffold for culture and transplantation of a pancreatic organoid in the form of a hydrogel with the lyophilized PEM.

In an embodiment of the present disclosure, in the process (4), the lyophilized PEM may be dissolved in a pepsin solution and a pH of the solution may be adjusted to form a hydrogel.

Yet another aspect of the present disclosure provides a method of culturing a pancreatic organoid in the above-described scaffold or a scaffold prepared by the above-described preparation method.

### EFFECTS OF THE INVENTION

A scaffold for culture and transplantation of a pancreatic organoid according to the present disclosure was developed as a new pancreatic organoid culture scaffold that overcomes the limitations of Matrigel, which is a representative conventional organoid culture scaffold, and is expected to be used as an elemental technology for various preclinical and clinical studies such as a large-scale drug screening platform based on pancreatic organoids or cell therapeutic agent for tissue regeneration, to create high added value in industrial and economic terms, and to promote the development of new medical industries.

By using the scaffold for culture and transplantation of a pancreatic organoid according to the present disclosure, it is possible to produce an advanced pancreatic organoid compared to that of conventional culture methods. Therefore, it can be used as a more economical and more accurate platform than conventional in vitro models for drug testing. Thus, an advanced pancreatic organoid-based in vitro model platform can greatly increase the success rate of new drug development and significantly reduce costs and lead time. Therefore, it is expected to greatly contribute to the advancement of the medical industry.

The decellularized pancreatic tissue-derived artificial matrix scaffold is expected to be widely used in various fields, such as disease modeling research that implements various intractable pancreatic diseases (acute and chronic pancreatitis, diabetes, pancreatic cancer, etc.) in vitro and investigate the mechanisms thereof, and establishment of a transplantation treatment platform. Since the prevalence of such intractable pancreatic diseases has increased significantly in recent years, a lot of research is needed. Therefore, the decellularized pancreatic tissue-derived artificial matrix scaffold can create profits as a research reagent.

The pancreatic organoid can be used for research on diabetes whose prevalence worldwide is 10% or more and which causes various complications and can also be used for research on pancreatic cancer, which has the lowest survival rate of all cancers. Thus, the pancreatic organoid can be of great value.

The scaffold according to the present disclosure can be applied to culture of tissue stem cell- and pancreatic cancer-derived pancreatic organoids and thus can contribute to the construction of a disease model customized for intractable disease and cancer patient and can also be used as a precision medicine platform technology. Further, it is expected to create enormous added value in consideration of the size of the precision medicine market that has been rapidly increasing in recent years.

Overall, as described above, the artificial scaffold according to the present disclosure is verified to have advantages in terms of safety and cost, showing better functionality as a culture system than Matrigel required for the application of a pancreatic organoid. Therefore, it is expected to replace Matrigel and create huge economic profits.

### BRIEF DESCRIPTION OF THE DRAWINGS

**FIG. 1a** shows a process of fabricating a decellularized pancreatic tissue-derived scaffold (pancreas extracellular matrix: PEM) for pancreatic organoid culture, and the fabricated PEM.
**FIG. 1b** shows a process of fabricating a decellularized pancreatic tissue-derived scaffold (pancreas extracellular matrix: PEM) for pancreatic organoid culture, and the fabricated PEM.
**FIG. 1c** shows a process of fabricating a decellularized pancreatic tissue-derived scaffold (pancreas extracellular matrix: PEM) for pancreatic organoid culture, and the fabricated PEM.
**FIG. 2a** shows the result of analyzing the PEM for pancreatic organoid culture.
**FIG. 2b** shows the result of analyzing the PEM for pancreatic organoid culture.
**FIG. 2c** shows the result of analyzing the PEM for pancreatic organoid culture.
**FIG. 2d** shows the result of analyzing the PEM for pancreatic organoid culture.
**FIG. 3** shows the analysis of mechanical properties of the PEM depending on concentration.
**FIG. 4a** shows the proteomic analysis of the PEM.
**FIG. 4b** shows the proteomic analysis of the PEM.
**FIG. 4c** shows the proteomic analysis of the PEM.
**FIG. 5a** shows the proteomic analysis of the PEM.
**FIG. 5b** shows the proteomic analysis of the PEM.
**FIG. 5c** shows the proteomic analysis of the PEM.
**FIG. 6a** shows the analysis of differences in mouse pancreatic organoid formation and differentiation potency of the PEM depending on concentration.
**FIG. 6b** shows the analysis of differences in mouse pancreatic organoid formation potency of the PEM depending on concentration.
**FIG. 6c** shows the analysis of differences in mouse pancreatic organoid differentiation potency of the PEM depending on concentration.
**FIG. 7** compares the growth of pancreatic organoids formed in the PEM and in a conventional scaffold (MAT).
**FIG. 8** shows the result of analyzing a pancreatic organoid cultured in the PEM.
**FIG. 9a** verifies the effect of enhancing the differentiation of a pancreatic organoid using the PEM.
**FIG. 9b** verifies the effect of enhancing the differentiation of a pancreatic organoid using the PEM.
**FIG. 10a** verifies the possibility of long-term culture of a pancreatic organoid using the PEM.
**FIG. 10b** verifies the possibility of long-term culture of a pancreatic organoid using the PEM.
**FIG. 11a** shows the result of verifying the possibility of long-term storage of the PEM.
**FIG. 11b** shows the result of verifying the possibility of long-term storage of the PEM.
**FIG. 12a** shows the result of verifying the possibility of long-term storage of the PEM.
**FIG. 12b** shows the result of verifying the possibility of long-term storage of the PEM.
**FIG. 13a** shows the culture of a human-induced pluripotent stem cell (hiPSC)-derived pancreatic organoid using the PEM.
**FIG. 13b** shows the culture of a human-induced pluripotent stem cell (hiPSC)-derived pancreatic organoid using the PEM.
**FIG. 14** shows the result of analyzing the hiPSC-derived pancreatic organoid cultured by using the PEM.
**FIG. 15a** verifies the possibility of long-term culture of the hiPSC-derived pancreatic organoid using the PEM.
**FIG. 15b** verifies the possibility of long-term culture of the hiPSC-derived pancreatic organoid using the PEM.
**FIG. 16a** shows the result of verifying the tissue-specific effect of the PEM for culture of the hiPSC-derived pancreatic organoid.
**FIG. 16b** shows the result of verifying the tissue-specific effect of the PEM for culture of the hiPSC-derived pancreatic organoid.
**FIG. 17** shows the result of culture of a pancreatic cancer organoid using the PEM.
**FIG. 18** verifies the possibility of the PEM to be used as a material for transplantation.
**FIG. 19a** shows the result of in vivo transplantation of a pancreatic organoid using the PEM.
**FIG. 19b** shows the result of in vivo transplantation of a pancreatic organoid using the PEM.
**FIG. 20** shows the result of in vivo transplantation of a pancreatic organoid using the PEM.
**FIG. 21** shows the result of in vivo transplantation of a pancreatic organoid using the PEM.

### BEST MODE FOR CARRYING OUT THE INVENTION

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operation and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless otherwise defined herein, all technical and scientific terms used herein have the same meaning as commonly understood by a person with ordinary skill in the art to which this disclosure belongs.

Various scientific dictionaries that include the terms included herein are well known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

An aspect of the present disclosure provides a scaffold for culture and transplantation of a pancreatic organoid, containing a pancreas extracellular matrix (PEM).

The term "extracellular matrix" refers to a natural scaffold for cell growth that is prepared by decellularization of tissue found in mammals and multicellular organisms. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various living tissues may differ in their overall structure and composition due to the unique role needed for each tissue.

The term "derive" or "derived" refers to a component obtained from any stated source by any useful method.

In an embodiment of the present disclosure, the PEM may be prepared from a pancreatic tissue decellularized by treatment with a mixed solution of Triton X-100 and ammonium hydroxide.

In an embodiment of the present disclosure, a concentration of the PEM in the scaffold may be from 1 mg/ml to 10 mg/ml, specifically from 2 mg/ml to 8 mg/ml. The concentration of the PEM may be, for example, from 2 mg/ml to 8 mg/ml, from 2 mg/ml to 6 mg/ml, from 2 mg/ml to 4 mg/ml, from 4 mg/ml to 8 mg/ml, from 4 mg/ml to 6 mg/ml, or from 6 mg/ml to 8 mg/ml. In an example, the concentration of the PEM may be 2 mg/ml, 4 mg/ml, 6 mg/ml, or 8 mg/ml. The PEM contained with a concentration out of the above-described range may make it impossible to achieve the intended effect of the present disclosure.

The scaffold includes a three-dimensional hydrogel prepared based on the PEM obtained by decellularization, and can be effectively used for pancreatic organoid culture.

The decellularized pancreatic tissue contains actual tissue-specific extracellular matrix components and thus can provide the physical, mechanical and biochemical environment of the tissue, and is highly efficient in enhancing differentiation into pancreatic tissue cells and tissue-specific functionality.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the intrinsic physiological properties of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions and to have an organ-like form with functionality and a tissue-specific function.

Another aspect of the present disclosure provides a method of preparing a scaffold for culture and transplantation of a pancreatic organoid, including: a process (1) of crushing an isolated pancreatic tissue; and a process (2) of treating the crushed pancreatic tissue with Triton X-100 and ammonium hydroxide for decellularization to prepare a decellularized PEM.

The process (1) is a process of crushing an isolated pancreatic tissue, and the pancreatic tissue may be isolated from a known animal. Examples of the animal may include cattle, pigs, monkeys, humans, etc. Also, in the present disclosure, the isolated pancreatic tissue is crushed and then decellularized, which results in high efficiency in decellularization. The isolated pancreatic tissue may be crushed by a known method. According to the present disclosure, the pancreatic tissue was crushed and decellularized, and, thus, the cells can be removed more efficiently at a higher level.

The process (2) is a process of treating the crushed pancreatic tissue with Triton X-100 and ammonium hydroxide for decellularization to prepare a decellularized PEM. According to the method of the present disclosure unlike a conventional decellularization method, the tissue is treated with Triton X-100 and ammonium hydroxide only to minimize damage to the tissue, and, thus, it is possible to preserve various proteins more in the pancreatic tissue. For example, the decellularization may be performed by stirring the crushed pancreatic tissue with Triton X-100 and ammonium hydroxide.

In an embodiment of the present disclosure, the method may further include, after the process (2), a process (3) of lyophilizing the decellularized PEM to prepare a lyophilized PEM.

The process (3) is a process of lyophilizing the decellularized PEM to prepare a lyophilized PEM. After drying, the lyophilized PEM may be exposed to electron beam, gamma radiation, ethylene oxide gas, or supercritical carbon dioxide for sterilization.

In an embodiment of the present disclosure, the method may further include, after the process (3), a process (4) of forming a scaffold for culture and transplantation of a pancreatic organoid in the form of a hydrogel with the lyophilized PEM.

The process (4) is a process of forming a scaffold for culture and transplantation of a pancreatic organoid in the form of a hydrogel with the lyophilized PEM. The process (4) may be performed through gelation. Specifically, the lyophilized PEM may be dissolved in a pepsin solution and a pH of the solution may be adjusted to form a hydrogel. The decellularized PEM may be crosslinked to prepare a three-dimensional hydrogel-type scaffold, and the gelated scaffold can be used in various ways in the fields related to tests, screening and organoid culture.

The term "hydrogel" is a material in which a liquid that contains water as a dispersion medium is hardened through a sol-gel phase transition to lose fluidity and to form a porous structure. The hydrogel can be formed by causing a hydrophilic polymer that has a three-dimensional network structure and a microcrystalline structure to contain water and to be expanded.

The gelation may be performed at a temperature of 37°C for 30 minutes after dissolving the lyophilized PEM in an acidic solution with a protease such as pepsin or trypsin and adjusting a pH, specifically setting a neutral pH in an electrolyte state of 1X PBS buffer by using 10X PBS and 1 M NaOH.

Yet another aspect of the present disclosure provides a method of culturing a pancreatic organoid in the above-described scaffold or a scaffold prepared by the above-described preparation method.

The conventional Matrigel-based culture system is an extract derived from animal cancer tissue, has a large difference between the batches, cannot mimic the actual pancreatic environment, and exhibits insufficient efficiency in differentiation or development into a pancreatic organoid. However, the scaffold can create a pancreatic tissue-like environment and thus is suitable for pancreatic organoid culture.

The culture refers to a process of maintaining and growing cells under suitable conditions, and the suitable conditions may refer to, for example, the temperature, nutrient availability, atmospheric CO₂ level and cell density at which the cells are maintained.

Appropriate culture conditions for maintaining, proliferating, expanding and differentiating different types of cells are known in the art and are documented. Suitable conditions for formation of the organoid may facilitate or allow cell differentiation and formation of a multicellular structure.

### MODE FOR CARRYING OUT THE INVENTION

The present disclosure is conceived to prepare a decellularized pancreatic tissue in which all of cell components are removed through decellularization of a pancreatic tissue and pancreas-specific extracellular matrix components remain and apply a three-dimensional hydrogel based on the decellularized pancreatic tissue to pancreatic organoid culture.

The PEM according to the present disclosure is composed of pure extracellular matrix components from which cellular antigens have been removed. Thus, it does not cause inflammatory reaction and immune rejection of tissue during transplantation and has excellent biocompatibility. The PEM is easy to produce and cheap. Therefore, it can be applied as a culture and transplantation material with higher economic efficiency and higher safety than Matrigel.

Through proteomics, it was confirmed that the PEM contains various extracellular matrices and factors specific to pancreatic tissue. It was confirmed that a stem cell-derived pancreatic organoid can be generated and grown in a prepared PEM. Also, the decellularized pancreatic tissue scaffold was tested at various concentrations and the optimal hydrogel concentration for pancreatic organoid culture was selected.

When the pancreatic organoid cultured in the developed PEM was compared to an organoid cultured in a control group, Matrigel, it was confirmed that the differentiation ability was similarly maintained or improved compared to the organoid cultured in the control group, Matrigel. Accordingly, it was verified that the PEM can replace the conventional Matrigel for pancreatic organoid culture. According to this series of results, it was confirmed that the pancreatic organoid cultured in the scaffold according to the present disclosure can better implement the structure and function of an actual pancreatic tissue than the organoid cultured in the conventional matrix (Matrigel).

Hereinafter, Examples will be presented for understanding of the present disclosure. However, the following Examples are illustrative only for better understanding of the present disclosure but do not limit the present disclosure.

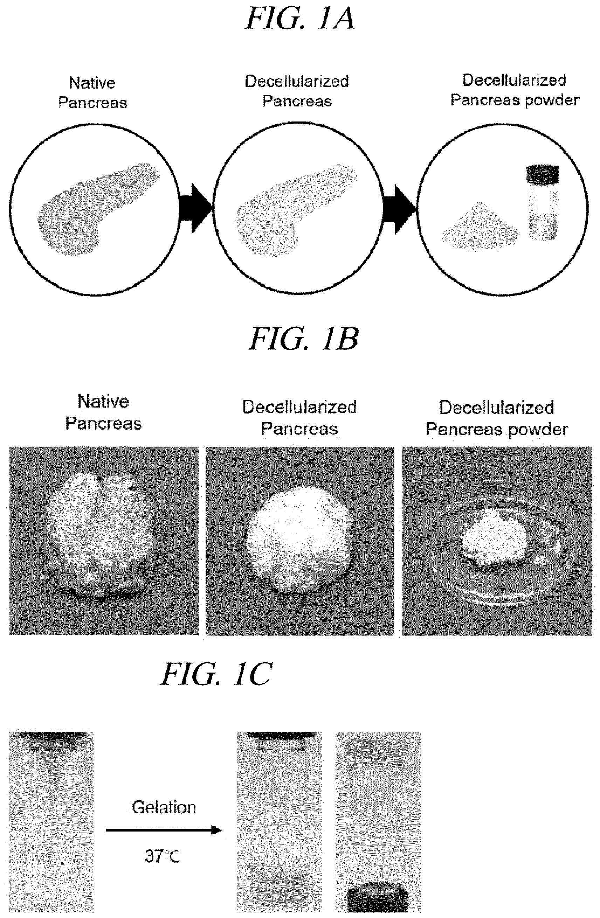

### Fabrication of pancreas extracellular matrix (PEM) for pancreatic organoid culture (FIG. 1)

A pancreas extracellular matrix (PEM) was fabricated from a porcine pancreatic tissue to prepare a culture scaffold for pancreatic organoid culture. It was confirmed that the present decellularization is performed using only a mixed solution of Triton X-100 and ammonium hydroxide unlike conventional methods to minimize damage to the tissue, and, thus, it is possible to preserve various proteins more in the pancreatic tissue. According to the present disclosure, the pancreatic tissue was finely cut and then decellularized, and, thus, the cells were more effectively and completely removed.

Specifically, **FIG. 1A** shows a schematic diagram showing a process of fabricating the PEM for pancreatic organoid culture.

(B) After the porcine pancreatic tissue was finely cut, all the cellular components in the tissue were removed through a chemical treatment with a Triton X-100 solution and an ammonium hydroxide solution and then lyophilized to obtain the PEM in the form of powder.

(C) 10 mg of the lyophilized PEM in the form of powder was treated with a 4 mg/ml pepsin solution (a solution of pepsin powder (4 mg) dissolved in 0.02 M HCl (1 ml)) and dissolved in a rocker at 240 rpm for 48 hours to carry out a solution process. After 10X PBS and 1M NaOH were added to set a neutral pH, gelation was induced at 37°C for 30 minutes. The fabricated three-dimensional hydrogel was used as a scaffold for pancreatic organoid culture.

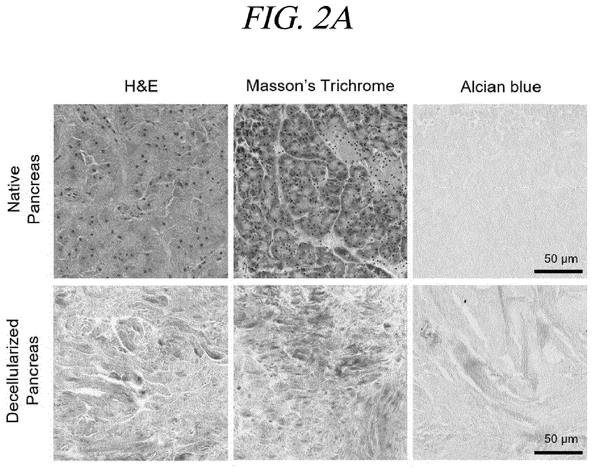

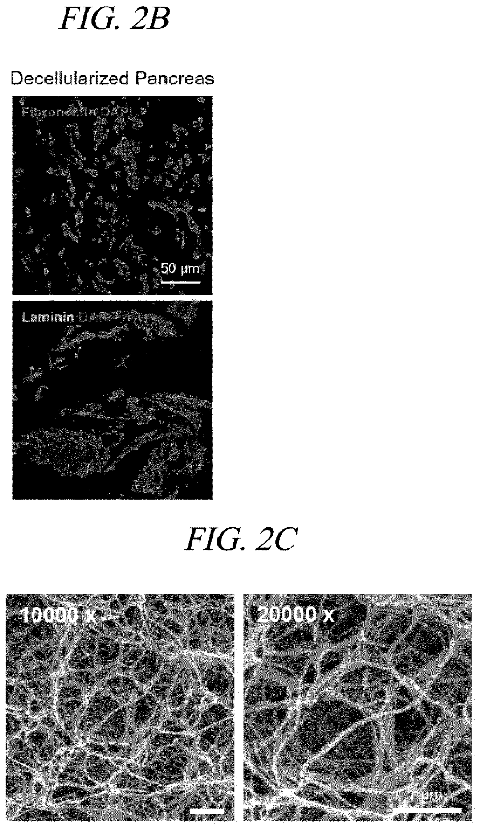

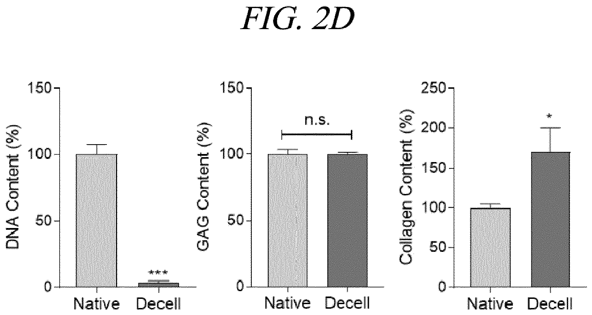

### Analysis of PEM for pancreatic organoid culture (FIG. 2)

(A) H&E staining was performed to confirm that all the cellular components in the PEM were removed, and Masson's trichrome staining was performed to confirm that the collagen components were well maintained even after the decellularization process. Further, Alcian blue staining was performed to confirm that the glycosaminoglycans were well preserved in the PEM.
(B) Immunostaining was performed to confirm whether ECM proteins such as fibronectin, laminin, etc. are well preserved in the pancreatic tissue after the decellularization process. As a result, it was confirmed that the ECM proteins such as fibronectin, laminin, etc. were well preserved in the PEM.
(C) Through scanning electron microscopy (SEM), it was confirmed that the PEM had a porous internal structure in the form of a nanofiber bundle. Therefore, it was confirmed that the PEM can provide a three-dimensional microenvironment suitable for pancreatic organoid culture.
(D) It was confirmed that most of the cellular components were removed by decellularization through quantitative comparison of DNAs before and after decellularization. Through quantitative analysis of glycosaminoglycans (GAGs) as a representative extracellular matrix component, it was confirmed that the GAGs were well preserved in the decellularized pancreatic tissue. It was also confirmed that when the amount of collagen was quantified, the collagen was well preserved in the PEM.

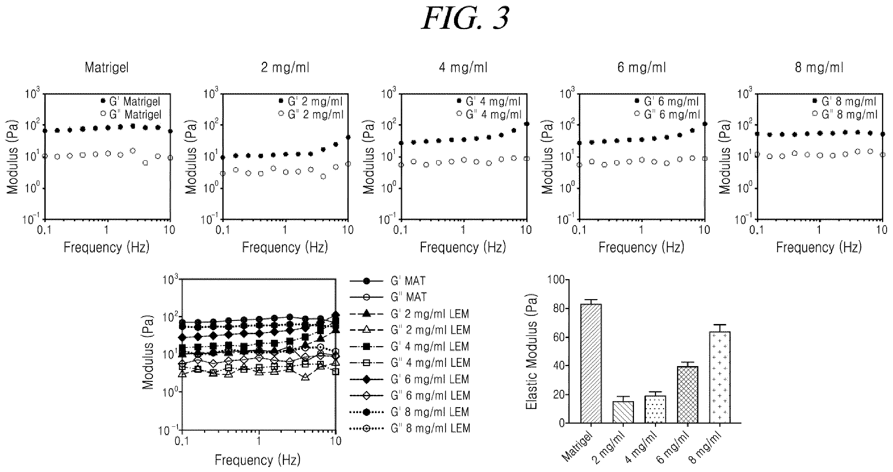

### Analysis of mechanical properties of PEM depending on concentration (FIG. 3)

In order to investigate the difference in mechanical properties of the PEM depending on concentration, the formation of PEM hydrogels was induced under four concentration conditions (2, 4, 6 and 8 mg/ml) and then the mechanical properties thereof were measured by rheological analysis. It was confirmed that a storage modulus G' value was consistently higher than a loss modulus G" value at all concentrations, and, thus, a stable polymer network was formed through crosslinking in the hydrogel. It was confirmed that as the PEM concentration increased, the properties also increased and were generally lower than those of the control group, Matrigel (MAT).

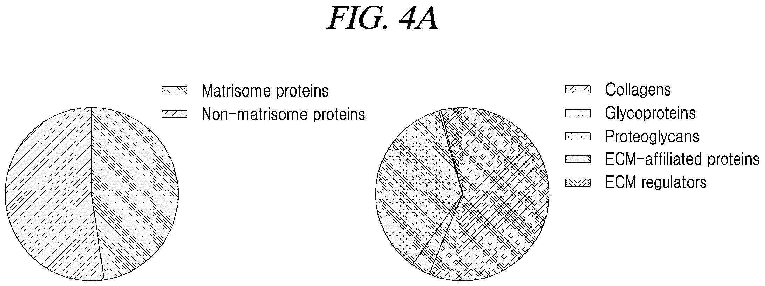

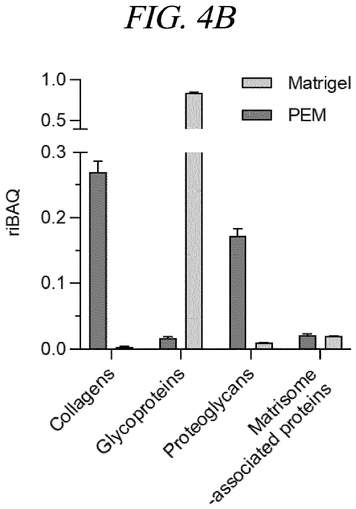

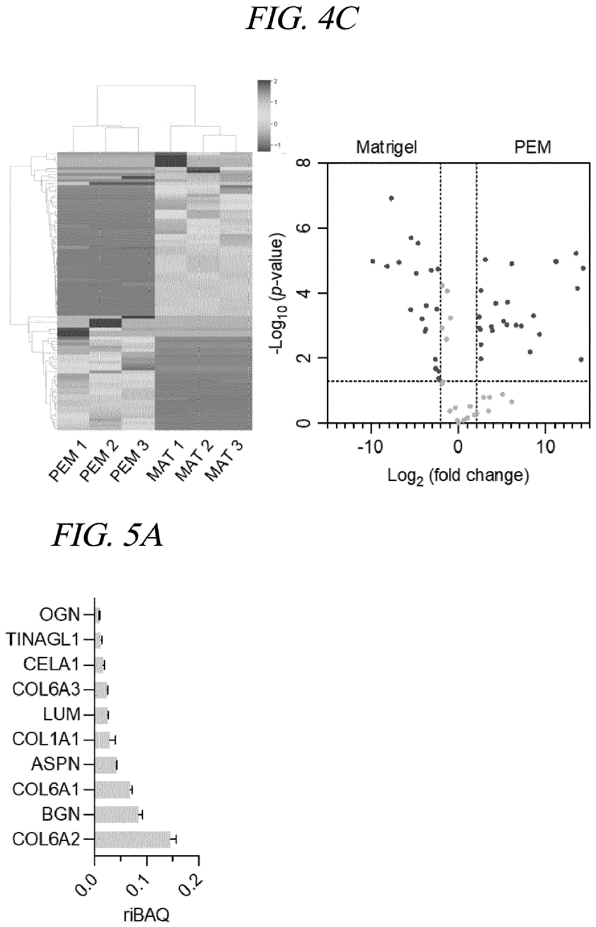

### Proteomic analysis of PEM (1) (FIG. 4)

(A) Proteomics was performed to identify the components of the PEM, and it was confirmed that various extracellular matrixes (collagens, glycoproteins, proteoglycans, etc.) specific to pancreatic tissue and growth factor proteins are contained in the PEM. As a result, it was confirmed that the ECM components forming the PEM are composed mainly of collagens and proteoglycans and further include ECM regulators and glycoproteins in this order.
(B) As a result of comparing the PEM to Matrigel, which is a conventionally commercialized scaffold, it was confirmed that Matrigel is composed mostly of glycoproteins, whereas the PEM is composed of various components such as collagens, proteoglycans, glycoproteins, etc.
(C) As a result of investigating and comparing the differences in protein expression between Matrigel and the PEM through a heat map and a volcano plot, it was confirmed from the heat map that there was a significant difference in expression level distribution of the all proteins between the two scaffolds. Also, as a result of identifying proteins expressed more significantly in each scaffold through the volcano plot, it was confirmed that the two scaffolds showed different expression patterns.

Accordingly, the PEM is composed of different extracellular matrix components from those of Matrigel, which is a conventionally commercialized organoid culture scaffold and is expected to better implement an in vivo pancreatic tissue microenvironment.

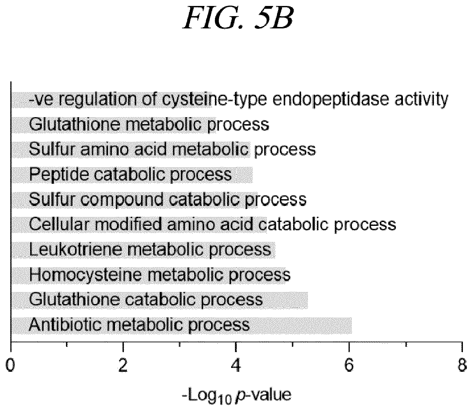

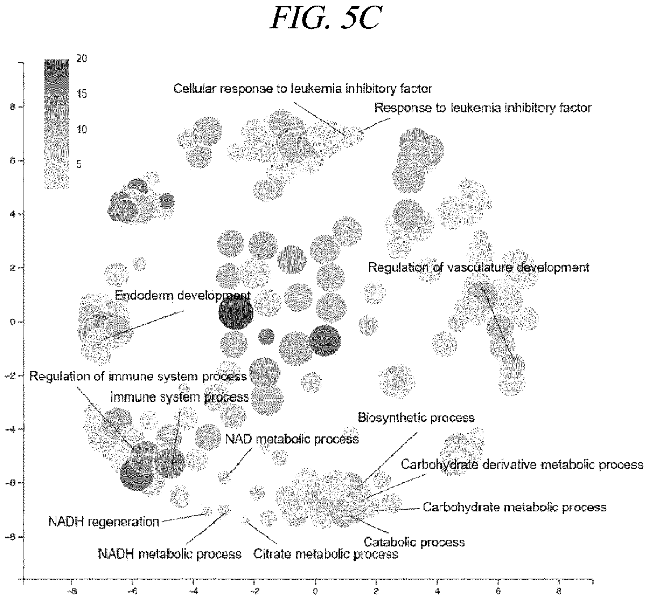

### Proteomic analysis of PEM (2) (FIG. 5)

(A) As a result of identifying top 10 ECM components contained in the PEM, it was confirmed that COL6A1 and COL6A2 that accounted for the largest portion are main components of the pancreatic islets of Langerhans and essential for survival of Langerhans cells, and biglycan (BGN), lumican (LUM), and asporin (ASPN) are ECM proteins important for development and structural formation of the pancreas. Accordingly, it was confirmed that the fabricated PEM contains various extracellular matrix proteins present in the actual pancreatic tissue and important for structure and function of the pancreas.
(B) When gene ontology analysis was performed to investigate the top 10 ECM components contained in the PEM, it was confirmed that these components have a function related to sulfur amino acid (SAA) important for metabolic regulation of pancreatitis and pancreatic cancer and a function related to glutathione metabolism essential for self-regeneration of pancreatic stem cells and thus play a vital role in function, differentiation and development of the pancreas.
(C) Even when gene ontology analysis was performed on all the components of the PEM, it was confirmed that they mainly play a role in immune system process or carbohydrate metabolic process and NAD/NADH metabolic process related to function, differentiation and development of the pancreas like the top 10 ECM components analyzed by gene ontology.

Therefore, it is considered that the PEM fabricated through decellularization facilitates efficient pancreatic organoid culture.

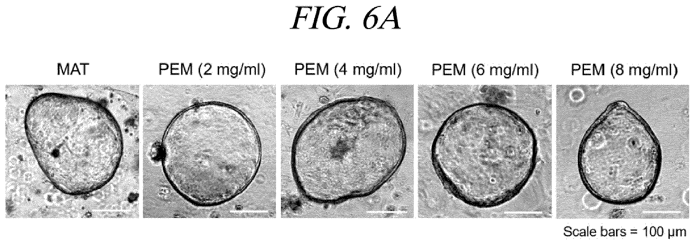

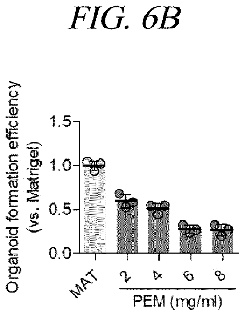

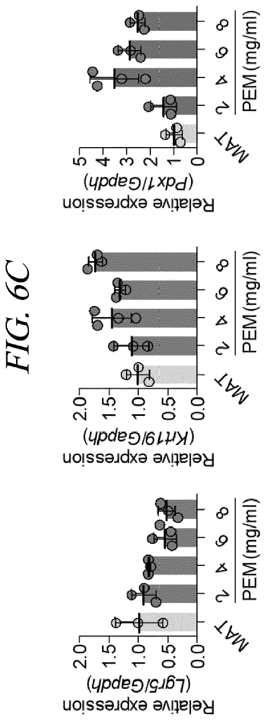

### Analysis of differences in mouse pancreatic organoid formation and differentiation potency of PEM depending on concentration (FIG. 6)

A PEM was fabricated for each concentration and applied to a mouse pancreatic organoid, and the expression levels of pancreas differentiation-related genes of organoids cultured for 7 days under respective concentration conditions were compared by quantitative PCR (qPCR) analysis. The commercialized culture scaffold, Matrigel (MAT), was used as a control group.
(A) It was confirmed that when the PEM hydrogels for pancreatic organoid culture were applied under various PEM concentration conditions, pancreatic organoids were well formed at any concentration (2, 4, 6, 8 mg/ml) with a similar morphology to that in Matrigel (MAT), which is the control group.
(B) It was confirmed that when the pancreatic organoid formation efficiencies of the PEMs depending on concentration were compared on day 7 of culture, the highest formation efficiencies were observed at 2 mg/ml and 4 mg/ml.
(C) When the gene expression levels depending on a PEM concentration were compared, Lgr5, a gene related to stemness, exhibited a similar or slightly decreased expression level in the pancreatic organoids cultured in the PEM hydrogels, but pancreas differentiation-related markers Krt19 and Pdx1 exhibited a tendency to increase the expression level. The 4 mg/ml PEM hydrogel was not much different from Matrigel in pancreatic organoid formation efficiency and showed higher expression levels of the pancreas differentiation-related markers. Thus, the PEM hydrogel was determined to have a PEM concentration of 4 mg/ml, which was applied later to pancreatic organoid culture.

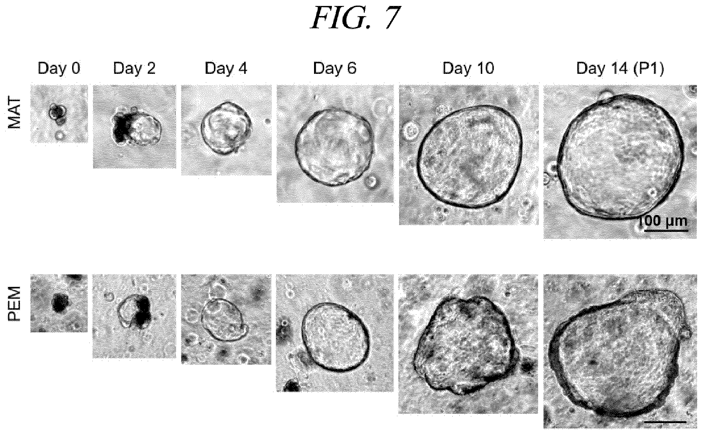

### Comparison in growth of pancreatic organoids cultured in PEM and conventional culture scaffold (MAT) (FIG. 7)

The growth rates of mouse pancreatic ductal cell-derived pancreatic organoids cultured in Matrigel, which is the most widely used scaffold for organoid culture, and the PEM with the optimal concentration of 4 mg/ml were compared. It was confirmed that the pancreatic organoids formed in the respective scaffolds started to form in the pancreatic duct and gradually increased in size, which means they were well cultured. It was confirmed that the pancreatic organoid cultured in the PEM hydrogel grew with a similar morphology at a similar rate to that of the organoid cultured in Matrigel.

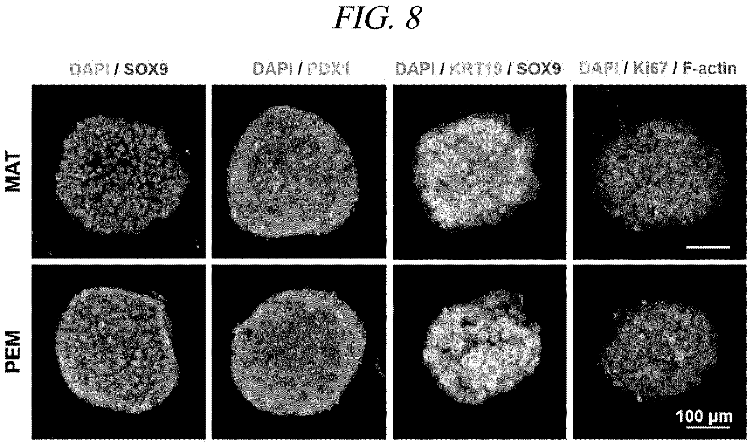

### Analysis of pancreatic organoid cultured in PEM (FIG. 8)

The pancreatic organoids cultured in Matrigel, which is the most widely used scaffold for organoid culture, and the PEM (4 mg/ml) were immunostained. The pancreatic organoids were prepared using pancreatic ductal cells extracted from the mouse pancreatic tissue and compared by immunostaining on day 7 of culture.

Immunostaining of the pancreas tissue-specific markers confirmed that the pancreas tissue-specific markers were well expressed in the pancreatic organoid cultured in the PEM at a similar level to that in the pancreatic organoid cultured in Matrigel, which is the control group; KRT19 (pancreatic duct marker), SOX9 (pancreatic duct progenitor marker), PDX1 (pancreatic endoderm marker).

Also, Ki67 (proliferative cell marker) was expressed at a similar level in the organoids of the two scaffold groups (PEM, Matrigel).

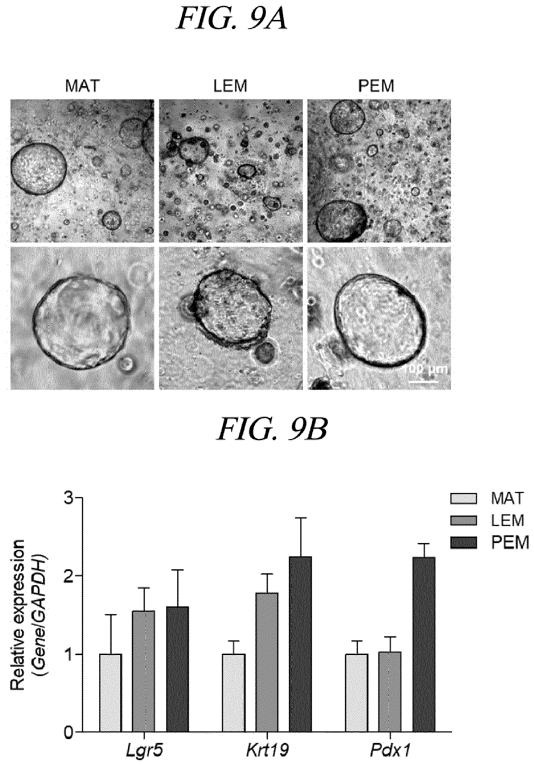

### Verification on effect of enhancing differentiation of pancreatic organoid using PEM (FIG. 9)

To verify that the decellularized PEM hydrogel can replace Matrigel, which is a conventional culture scaffold and has a tissue-specific functionality in enhancing the differentiation of an organoid through a pancreas-specific microenvironment, pancreatic organoids cultured in the decellularized PEM hydrogel and another organ (liver) tissue-derived extracellular matrix (LEM) hydrogel were compared in terms of differentiation potency. A Matrigel group was used as a control group.
(A) To verify the effect of the PEM in enhancing differentiation, a pancreatic organoid was also cultured in the LEM group as in the PEM group. It was confirmed that the organoids were well formed to have a normal morphology in all of the culture scaffolds.
(B) As a result of quantitative comparison in terms of gene expression of markers related to stemness or pancreas differentiation by qPCR analysis, it was confirmed that the stemness-related gene exhibited a similar or slightly increased expression level, but the pancreas differentiation markers Krt19 and Pdx1 exhibited the most significant increase in the pancreatic tissue-specific PEM group.

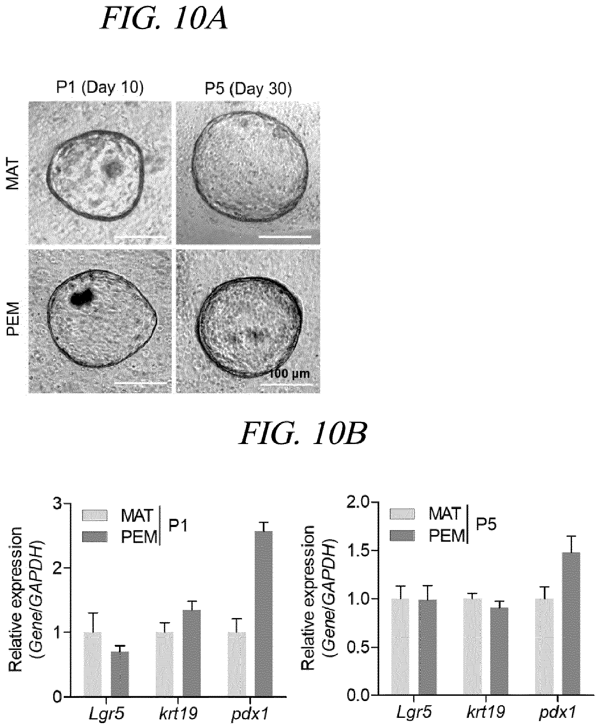

### Verification on possibility of long-term culture of pancreatic organoid using PEM (FIG. 10)

Long-term culture of a pancreatic organoid in the previously established PEM was attempted, and expression of a pancreas specific-differentiation marker was analyzed. A Matrigel group (MAT) was used as a control group.
(A) It was confirmed that even when the pancreatic organoid was cultured for 1 month or more with continuous subculture in the PEM (passage 5), the pancreatic organoid was well cultured to have a similar morphology with a similar size to the pancreatic organoid cultured in Matrigel.
(B) As a result of comparing the expression levels of the stemness marker and the pancreas differentiation marker on day 10 (P1) and day 30 (P5) of pancreatic organoid culture, it was confirmed that the stemness gene exhibited a similar expression level and the pancreas differentiation marker exhibited a higher expression level in the pancreatic organoid cultured in the PEM compared to the organoid cultured in Matrigel. The differentiation marker exhibited a continuous higher expression level in the pancreatic organoid cultured in the PEM for 30 days than in the organoid in the Matrigel group, which verifies the possibility of long-term culture of the pancreatic organoid using the PEM.

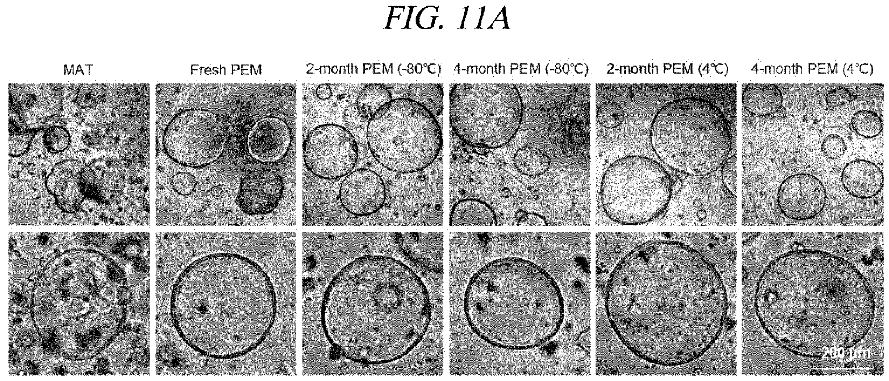

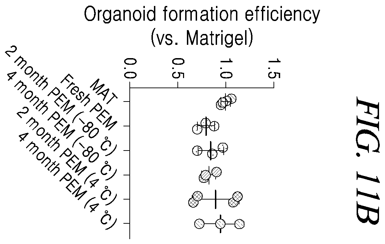

### Verification on possibility of long-term storage of PEM (1) (FIG. 11)

After a PEM solution was cryopreserved at -80°C and cold-stored at 4°C for a long time and then thawed and used for pancreatic organoid culture. Matrigel was used as a control group. On day 7 of culture, optical microscopy images and formation efficiencies were compared.
(A) It was confirmed that pancreatic organoids were well formed in all of the matrix groups including the control group, Matrigel, a PEM hydrogel prepared immediately before organoid culture, and a PEM hydrogel cryopreserved at -80°C and cold-stored at 4°C for a long time, respectively.
(B) On day 7 of culture, the pancreatic organoid formation efficiencies were compared. The pancreatic organoids were well formed even in the hydrogel prepared using the PEM solution cryopreserved and cold-stored for 2 months or 4 months with a similar formation efficiency to that in the PEM hydrogel prepared immediately before pancreatic organoid culture.

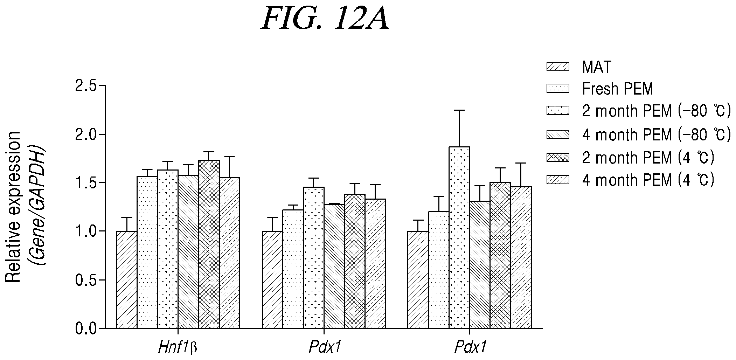

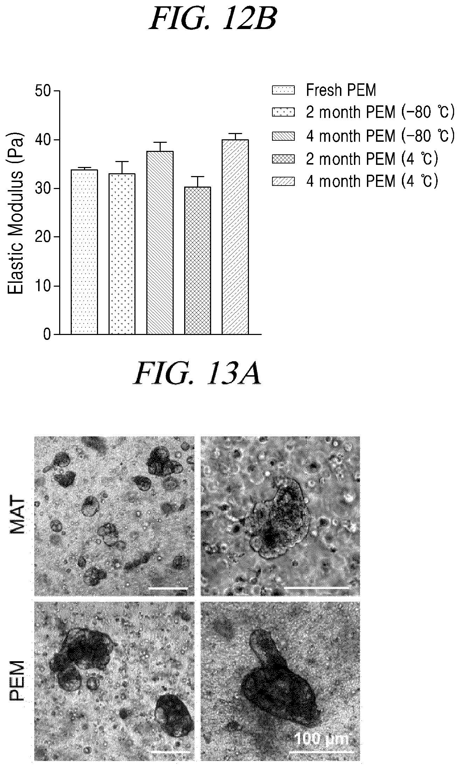

### Verification on possibility of long-term storage of PEM (2) (FIG. 12)

After a PEM solution was cryopreserved at -80°C and cold-stored at 4°C for a long time and then thawed and used for pancreatic organoid culture. Matrigel was used as a control group. On day 7 of culture, the gene expression levels of pancreas markers were compared by qPCR analysis.
(A) It was confirmed that when pancreatic organoids cultured in Matrigel (MAT), a PEM hydrogel prepared immediately before culture, and a hydrogel prepared using the PEM solution cryopreserved and cold-stored for 2 months or 4 months, respectively, were compared in terms of gene expression levels of pancreas markers, all the pancreas differentiation markers (Hnf1β, Pdx1 and Krt19) exhibited higher expression levels without a significant difference regardless of storage conditions in the PEM hydrogel group than in the Matrigel group.
(B) Rheological analysis was performed to measure the differences in properties between the PEM hydrogel prepared immediately before culture and the long-term stored PEM hydrogel. As a result, it was confirmed that even when the hydrogel was prepared using the PEM solution cryopreserved and cold-stored for a long time, there was little difference in mechanical properties and the hydrogel maintained an elastic modulus of 30 Pa to 40 Pa. Accordingly, it was confirmed that the PEM hydrogel is stably maintained without degradation even after long-term storage and thus can be used for pancreatic organoid culture.

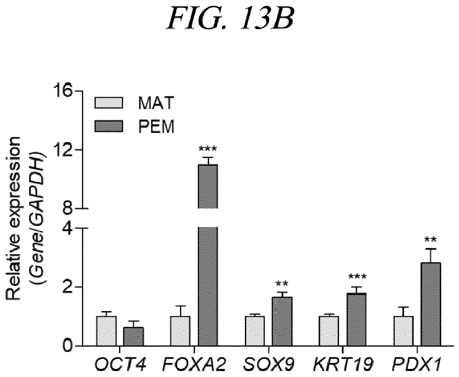

### Human-induced pluripotent stem cell (hiPSC)-derived pancreatic organoid culture using PEM (FIG. 13)

An hiPSC was differentiated into a pancreatic progenitor (PP) through a definitive endoderm (DE) and a pancreatic endoderm (PE) for pancreatic organoid formation and then encapsulated into a PEM hydrogel, followed by 3D culture. A Matrigel (MAT) group was used as a control group.
(A) It was confirmed that when the hiPSC-derived pancreatic precursor cell was cultured in the PEM hydrogel (4 mg/ml) and MAT, a 3D organoid was formed in each of the two groups within 2 days.
(B) It was confirmed that when the gene expression levels of the pancreatic organoids cultured in the two groups for 10 days were compared by qPCR analysis, the expression level of OCT4, a pluripotent marker, slightly decreased in the PEM group and the expression levels of pancreas differentiation-related markers (FOXA2, SOX9, KRT19 and PDX1) significantly increased in the PEM group; OCT4 (pluripotency marker), FOXA2 (pancreatic β-cell differentiation marker), SOX9 (pancreatic duct progenitor marker), KRT19 (pancreatic duct marker), PDX1 (pancreatic endoderm marker). This result verifies that when a pancreatic organoid is cultured using a PEM hydrogel, the pancreas differentiation and maturity of the organoid can be improved compared to culture using Matrigel.

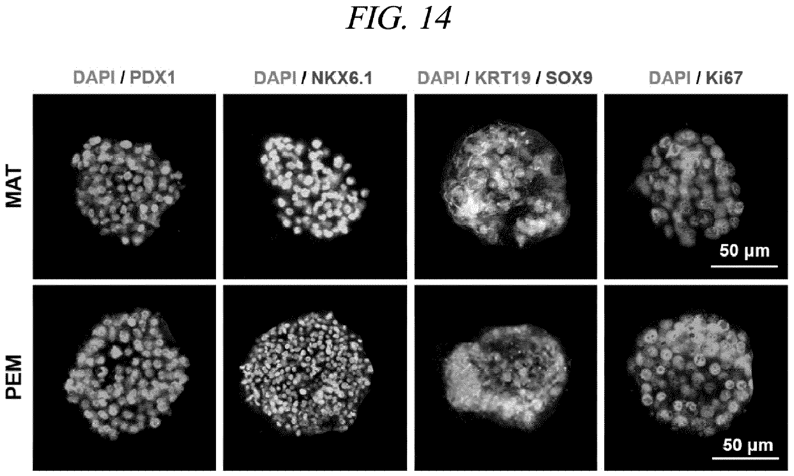

### Analysis of hiPSC-derived pancreatic organoid cultured by using PEM (FIG. 14)

The pancreatic organoids cultured in Matrigel (MAT) and the PEM hydrogel (4 mg/ml) were immunostained. The pancreatic organoids were prepared from the pancreatic precursor cell differentiated from the hiPSC, and compared by immunostaining on day 7 of organoid formation.

As a result of comparison with the pancreatic organoid cultured in the control group, Matrigel, by immunostaining of pancreatic tissue-specific markers, it was confirmed that the pancreatic tissue-specific markers were well expressed in the pancreatic organoid cultured in the PEM at a similar level to that in the pancreatic organoid cultured in the Matrigel group; KRT19 (pancreatic duct marker), SOX9 (pancreatic duct progenitor marker), PDX1 (pancreatic endoderm marker), NKX6.1 (pancreatic β-cell differentiation marker).

Ki67, a proliferative cell marker, was also observed at similar expression levels in the two scaffold groups (PEM and Matrigel).

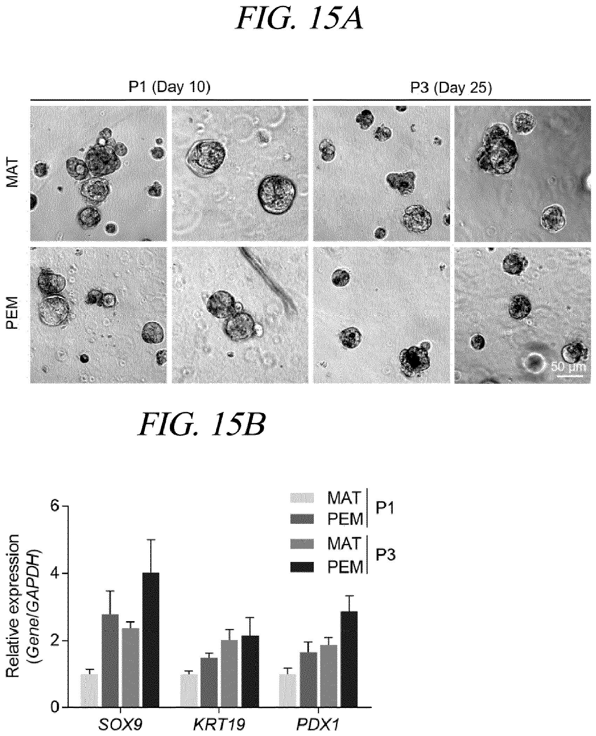

### Verification on possibility of long-term culture of hiPSC-derived pancreatic organoid using PEM (FIG. 15)

Long-term culture of a pancreatic organoid in the previously established PEM was attempted, and expression of a pancreas specific-differentiation marker was analyzed. A Matrigel group (MAT) was used as a control group.
(A) It was confirmed that even when the pancreatic organoid was cultured for 25 days or more with continuous subculture in the PEM, the pancreatic organoid was well cultured to have a similar morphology with a similar size to the pancreatic organoid cultured in Matrigel.
(B) It was confirmed that even when the pancreatic organoid was cultured for 25 days or more, the expression level of the pancreas differentiation marker increased in the pancreatic organoid cultured in the PEM compared to the organoid cultured in Matrigel. Also, it was confirmed that as the time period of culture increased, differentiation of the pancreatic organoid was further improved in the PEM hydrogel.

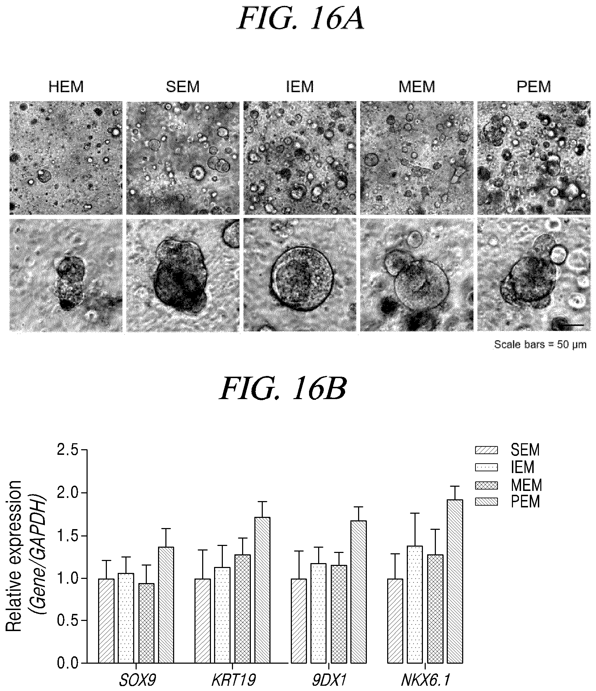

### Verification on tissue-specific effect of PEM for culture of hiPSC-derived pancreatic organoid (FIG. 16)

To verify that the pancreas-specific extracellular matrix components contained in the PEM have a tissue-specific effect on pancreatic organoid culture, pancreatic organoids were also cultured in decellularized scaffolds derived from other organs and subjected to comparison. After the pancreatic organoids were cultured for 5 days in the respective tissue-derived decellularized scaffolds, the gene expression levels were compared.
(A) As a result of culture using heart, stomach, intestine, muscle and pancreas-derived decellularized hydrogels, it was confirmed that a pancreatic organoid was not formed in the heart tissue-derived hydrogel, but formed in the other tissue-derived decellularized hydrogels.
(B) After culture for 5 days in each of the organ-derived decellularized scaffolds, the expression levels of the pancreatic organoid differentiation-related genes were compared by qPCR analysis. As a result of analysis of SOX9 and KRT19 related to pancreatic ductal cell differentiation, PDX1 related to pancreatic endoderm differentiation, and NKX6.1 related to pancreatic precursor cell development, it was confirmed that the expression levels thereof were the highest in the organoid cultured in the PEM. This result verifies that the PEM has a tissue-specific effect on pancreatic organoid culture.

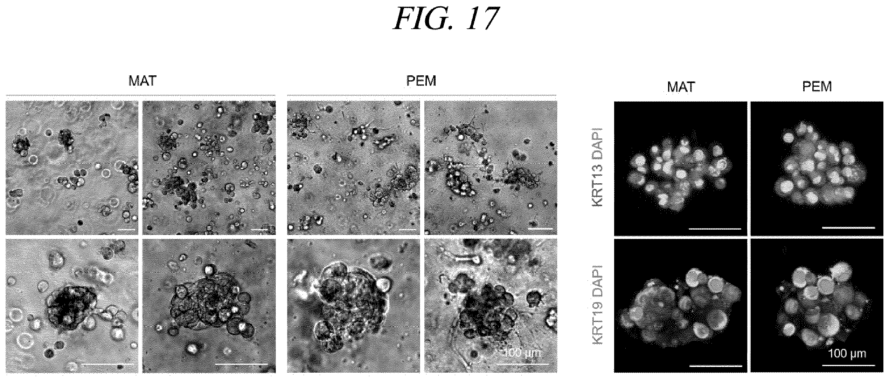

### Culture of pancreatic cancer organoid using PEM (FIG. 17)

The possibility of culturing a pancreatic cancer organoid using the PEM was checked. It was confirmed that when a human-induced PANC-1 pancreatic cancer cell line was three-dimensionally cultured in a PEM hydrogel, a cancer organoid was formed (organoid images and immunostaining analysis on day 5 of culture).

It was confirmed through optical microscopy that pancreatic cancer organoids were well formed in Matrigel and the PEM hydrogel. Also, KRT13 and KRT19, proteins overexpressed in exocrine pancreatic cancer, were detected through immunostaining, and, thus, it was confirmed that cells in the pancreatic cancer organoid proliferated actively and pancreatic cancer-related markers were well expressed.

**The** present test verified that the PEM can be used for culturing not only a stem cell-derived pancreatic organoid but also a pancreatic cancer organoid. Thus, it was confirmed that the PEM hydrogel can be applied as an elemental technology of a culture platform for the construction of an in vitro pancreatic cancer model.

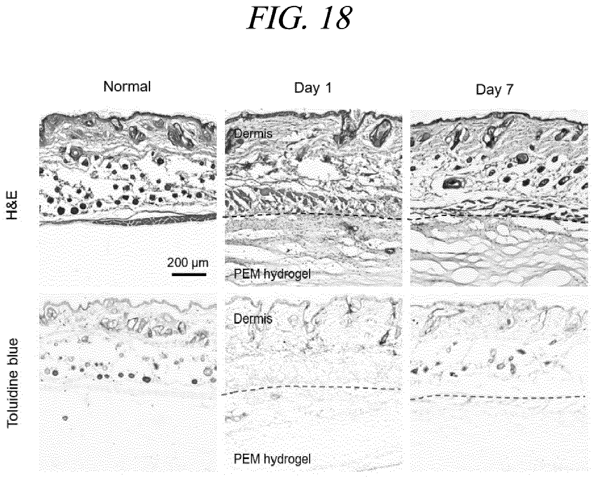

### Verification on possibility of PEM to be used as material for transplantation (FIG. 18)

To check whether transplantation of the PEM causes an immune response, the PEM hydrogel was subcutaneously injected into a mouse, the occurrence of an immune response and an inflammatory response was checked for a week.

As a result of comparison with the normal tissue by H&E staining, it was confirmed that any immune response and infiltration of immune cells did not occur in the PEM hydrogel-transplanted site and the dermis. Also, as a result of toluidine blue staining of mast cells infiltrating during transplantation, it was confirmed that the mast cells infiltrating for a week were not stained, and, thus, the PEM hydrogel did not cause an immune response during transplantation. Thus, it was confirmed that the PEM hydrogel can be used as a material for organoid transplantation.

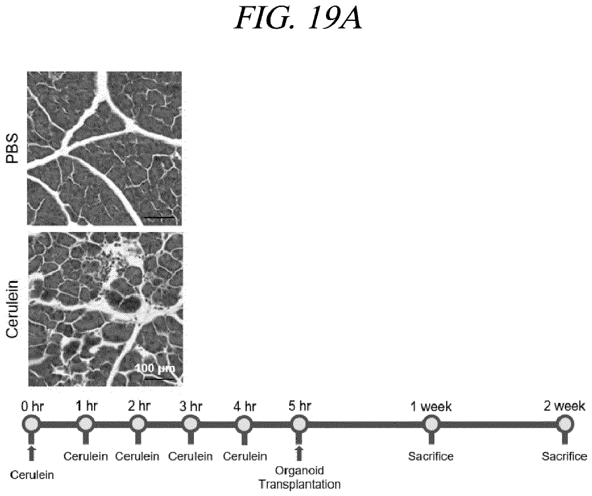

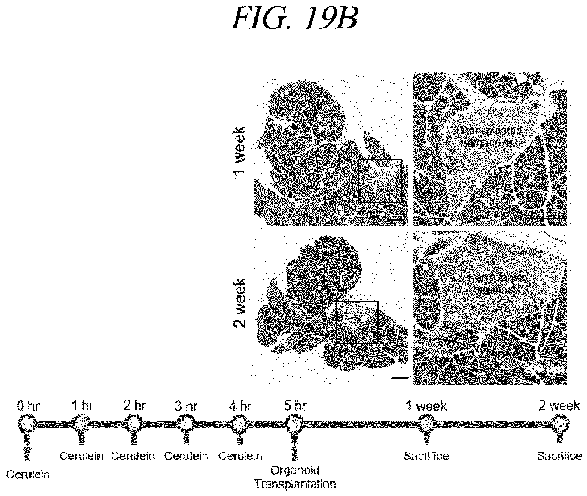

### In vivo transplantation of pancreatic organoid using PEM (1) (FIG. 19)

Animal tests were conducted to apply the PEM as a material for pancreatic organoid transplantation, and histological analysis was performed. For efficient pancreatic organoid delivery and engraftment into a damaged pancreatic tissue of an acute pancreatitis mouse model, 1000 to 1200 pancreatic organoids were transplanted using a 4 mg/ml PEM hydrogel. The viscosity of the hydrogel was adjusted for easy injection during transplantation, and the organoid was mixed into 50 µl of the PEM hydrogel and then transplanted for efficient organoid engraftment.
(A) A mouse was administered 40 µg/kg cerulein five times at 1-hour interval by intraperitoneal injection to prepare the acute pancreatitis mouse model, and H&E staining was performed to check the degree of pancreatitis. It was confirmed that vacuolization of cytoplasm and infiltration of immune cells, features of pancreatitis, occurred in the tissue with pancreatitis induced by injection of cerulein compared to the group injected with PBS.
(B) The pancreatic organoid was transplanted into the acute pancreatitis mouse model using the PEM hydrogel scaffold, and H&E staining was performed to observe the engraftment and tissue regeneration at week 1 of transplantation and week 2 of transplantation. It was confirmed that the organoid transplanted into the surface of the pancreatic tissue was well engrafted, and the transplantation site had a greater area and the transplanted organoid was better integrated with the existing tissue at week 2 than at week 1 due to the growth of the transplanted organoid.

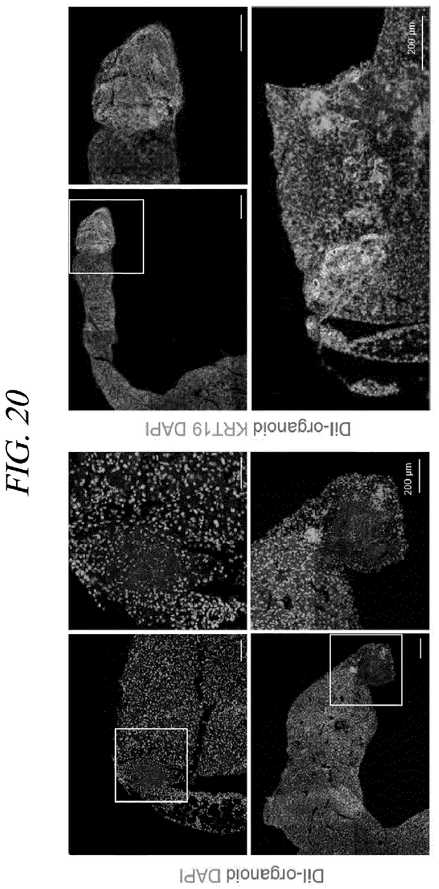

### In vivo transplantation of pancreatic organoid using PEM (2) (FIG. 20)

A pancreatic organoid labelled with a fluorescent dye Dil was transplanted into an acute pancreatitis mouse model using a PEM hydrogel scaffold, and the expression of fluorescence was observed to check the engraftment at week 1 of transplantation. It was confirmed that the pancreatic organoid transplanted into the damaged pancreatic tissue site was well engrafted for a week. Also, as a result of staining KRT19, a pancreatic duct marker, it was confirmed that KRT19 was well expressed in the pancreatic organoid having characteristics of the pancreatic duct as well as the pancreatic duct of the surrounding pancreatic tissue. No Dil fluorescence was observed in non-transplanted tissues.

This result shows that the PEM hydrogel can be applied as a material not only for pancreatic organoid culture but also for in vivo transplantation.

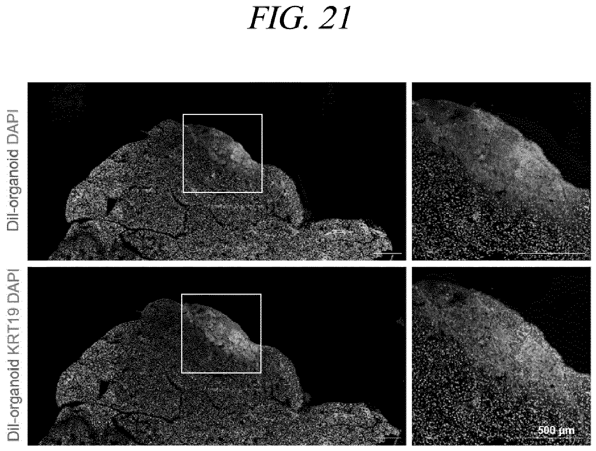

### In vivo transplantation of pancreatic organoid using PEM (3) (FIG. 21)

A pancreatic organoid labelled with a fluorescent dye Dil was transplanted into an acute pancreatitis mouse model using a PEM hydrogel scaffold, and the expression of fluorescence was observed to check the engraftment at week 2 of transplantation. It was confirmed that the pancreatic organoid transplanted into the damaged pancreatic tissue site was well engrafted for two weeks. Also, as a result of staining KRT19, a pancreatic duct marker, it was confirmed that KRT19 was well expressed in the pancreatic organoid having characteristics of the pancreatic duct as well as the pancreatic duct of the surrounding pancreatic tissue. No Dil fluorescence was observed in non-transplanted tissues.

This result shows that the PEM hydrogel can be applied as a material not only for pancreatic organoid culture but also for in vivo transplantation.

The above description of the present disclosure is provided for the purpose of illustration, and it would be understood by a person with ordinary skill in the art that various changes and modifications may be made without changing technical conception and essential features of the present disclosure. Thus, it is clear that the above-described examples are illustrative in all aspects and do not limit the present disclosure.

## Claims

1. A scaffold for culture and transplantation of a pancreatic organoid, containing a pancreas extracellular matrix (PEM).

2. The scaffold of Claim 1,
wherein the PEM is decellularized by treating a pancreatic tissue with a mixed solution of Triton X-100 and ammonium hydroxide.

3. The scaffold of Claim 1,
wherein a concentration of the PEM in the scaffold is from 1 mg/ml to 10 mg/ml.

4. A method of preparing a scaffold for culture and transplantation of a pancreatic organoid, comprising:
a process (1) of crushing an isolated pancreatic tissue; and
a process (2) of treating the crushed pancreatic tissue with Triton X-100 and ammonium hydroxide for decellularization to prepare a decellularized PEM.

5. The method of preparing a scaffold for culture and transplantation of a pancreatic organoid of Claim 4, further comprising:
after the process (2), a process (3) of lyophilizing the decellularized PEM to prepare a lyophilized PEM.

6. The method of preparing a scaffold for culture and transplantation of a pancreatic organoid of Claim 5, further comprising:
after the process (3), a process (4) of forming a scaffold for culture and transplantation of a pancreatic organoid in the form of a hydrogel with the lyophilized PEM.

7. The method of preparing a scaffold for culture and transplantation of a pancreatic organoid of Claim 6,
wherein in the process (4), the lyophilized PEM is dissolved in a pepsin solution and a pH of the solution is adjusted to form the hydrogel.

8. A method of culturing a pancreatic organoid in the scaffold of Claim 1 or a scaffold prepared by the preparation method of Claim 4.
